# EUROPÄISCHE PATENTSCHRIFT

(11) **EP 0 924 196 B1**
(45) Veröffentlichungstag und Bekanntmachung des Hinweises auf die Patenterteilung: **18.06.2003**
(21) Anmeldenummer: 98123418.0
(22) Anmeldetag: 09.12.1998
(51) Int. Cl.: C07C 253/30, C07D 211/02, C07C 211/64, C07B 37/04

(54) **Verfahren zur Alkylierung von Alkyl- oder Benzylcyanderivaten in Gegenwart von Trialkylaminen oder -phosphinen**
Process for the alkylation of alkyl- or benzonitrile in the presence of trialkyl amines or phosphines
Procédé pour l'alkylation de nitriles aliphatiques ou benzyliques en présence d'amines ou de phosphines trialkyées

(30) Priorität: 17.12.1997 DE 19756091; 29.01.1998 DE 19803408
(43) Veröffentlichungstag der Anmeldung: 23.06.1999
(73) Patentinhaber: Aventis Pharma Deutschland GmbH, 65929 Frankfurt am Main (DE)
(72) Erfinder: Korb, Gerhard Dr., 63512 Hainburg (DE); Flemming, Hans-Wolfram Dr., 61250 Usingen (DE); Lehnert, Rudolf Dr., 55122 Mainz (DE)

(56) Entgegenhaltungen:
- EP-A- 0 671 379
- DE-A- 2 206 778
- US-A- 3 668 255

## Beschreibung

In der Europäischen Patentanmeldung EP 0 671 379 wird ein Verfahren zur Methylierung von organischen Verbindungen in Anwesenheit von Trialkylaminen und Dimethylcarbonat beschrieben. Die Ausbeute an α,α-Dimethylbenzylcyanid liegt bei 29 %. Im US-Patent 3,668,255 wird ein Verfahren zur Alkylierung von aliphatischer Ketone in Anwesenheit von tertiären Amin und festen Alkalibasen beschrieben. Ferner wird die Alkylierung von Benzylcyanid mit Alkylierungsmitteln wie Methyljodid oder Methylchlorid in Anwesenheit von starken Basen wie Natriumhydrid, Natriumamid oder Natriumalko-holat beschrieben (Smith et al., J. Org. Chem. 36 (1971), 15, Seiten 2132-2137; Trivedi et al., J. Med. Chem., EN, 36, 22, (1993), Seiten 3300- 3307). Nachteil dieser Reaktion sind erhöhte Bildung von Etherprodukten, das Entstehen und die Emission von Wasserstoff und Ammoniak zusammen mit dem Alkylierungsmittel. Darüber hinaus müssen die starken Basen erst ökologisch und ökonomisch aufwendig hergestellt werden.

Aufgabe der vorliegenden Erfindung ist es ein Verfahren zu finden, durch das Verbindungen der Formel II in hohen Ausbeuten und Reinheit alkyliert werden.

Die Erfindung betrifft daher ein Verfahren zur Gewinnung der Verbindung der Formel I 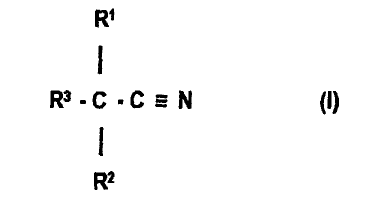 wobei
R¹ für
   1. (C₁-C₂₀)-Alkyl,
   2. (C₁-C₂₀)-Alkyl, welches ein-, zwei- oder dreifach substituiert ist durch
      2.1. (C₃-C₆)-Cycloalkyl,
      2.2. -OH,
      2.3. (C₁-C₆)-Alkyl-C(O)-O-,
      2.4. (C₁-C₆)-Alkyl-O-,
      2.5. (C₁-C₆)-Alkyl-O-(C₁-C₄)-Alkyl-O-,
      2.6. Halogen,
      2.7. -CF₃,
      2.8. -CN,
      2.9. -NO₂,
      2.10. HO-C(O)-,
      2.11. (C₁-C₆)-Alkyl-O-C(O)-,
      2.12. Methylendioxo,
      2.13. R⁵-(R⁶)N-C(O)-, worin R⁵ und R⁶ gleich oder verschieden sind und für Wasserstoffatom oder (C₁-C₆)-Alkyl- stehen,
      2.14. R⁵-(R⁶)N-, worin R⁵ und R⁶ gleich oder verschieden sind und für Wasserstoffatom oder (C₁-C₆)-Alkyl stehen, oder
      2.15. Phenyl, welches unsubstituiert oder ein-, zwei- oder dreifach unabhängig voneinander substituiert ist durch (C₁-C₆)-Alkyl oder wie unter 2.1. bis 2.14. beschrieben substituiert ist,
   3. (C₂-C₂₀)-Alkenyl,
   4. (C₂-C₂₀)-Alkenyl, welches ein-, zwei- oder dreifach unabhängig voneinander wie unter 2.1. bis 2.15. beschrieben substituiert ist, steht,
R² dieselbe Bedeutung wie R¹ hat oder
   für
   1. Phenyl oder
   2. Phenyl, welches ein-, zwei- oder dreifach substituiert ist durch
      2.1. (C₁-C₆)-Alkyl, worin die Alkylkette gerade oder verzweigt ist,
      2.2. (C₃-C₆)-Cycloalkyl,
      2.3. -OH,
      2.4. (C₁-C₆)-Alkyl-C(O)-O-,
      2.5. (C₁-C₆)-Alkyl-O-,
      2.6. (C₁-C₆)-Alkyl-O-(C₁-C₄)-Alkyl-O-,
      2.7. Halogen,
      2.8. -CF₃,
      2.9. -CN,
      2.10. -NO₂,
      2.11. HO-C(O)-,
      2.12. (C₁-C₆)-Alkyl-O-C(O)-,
      2.13. Methylendioxo,
      2.14. R⁵-(R⁶)N-C(O)-, worin R⁵ und R⁶ gleich oder verschieden sind und für Wasserstoffatom oder (C₁-C₆)-Alkyl- stehen, oder
      2.15. R⁵-(R⁶)N-, worin R⁵ und R⁶ gleich oder verschieden sind und für Wasserstoffatom oder (C₁-C₆)-Alkyl stehen, steht
oder R¹ und R² zusammen mit dem Kohlenstoffatom an das sie gebunden sind und den Resten R³ und -CN eine Verbindung der Formel IV bilden, 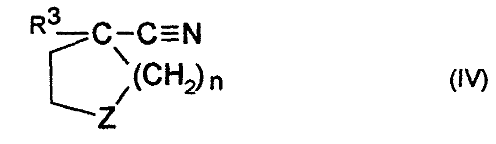 wobei
Z für N-, O- oder S-Atom steht und n 1 oder 2 ist, oder wenn Z für N-oder S-Atom steht, Z unsubstituiert oder durch R substituiert ist, worin
   R (C₁-C₆)-Alkyl, Benzyl oder Phenyl bedeutet,
R³ für
   1. Phenyl oder
   2. Phenyl, welches ein-, zwei- oder dreifach substituiert ist durch
      2.1. (C₁-C₆)-Alkyl, worin die Alkylkette gerade oder verzweigt ist,
      2.2. (C₃-C₆)-Cycloalkyl,
      2.3. -OH,
      2.4. (C₁-C₆)-Alkyl-C(O)-O-,
      2.5. (C₁-C₆)-Alkyl-O-,
      2.6. (C₁-C₆)-Alkyl-O-(C₁-C₄)-Alkyl-O-,
      2.7. Halogen,
      2.8. -CF₃,
      2.9. -CN,
      2.10. -NO₂,
      2.11. HO-C(O)-,
      2.12. (C₁-C₆)-Alkyl-O-C(O)-,
      2.13. Methylendioxo,
      2.14. R⁵-(R⁶)N-C(O)-, worin R⁵ und R⁶ gleich oder verschieden sind und für Wasserstoffatom oder (C₁-C₆)-Alkyl- stehen, oder
      2.15. R⁵-(R⁶)N-, worin R⁵ und R⁶ gleich oder verschieden sind und für Wasserstoffatom oder (C₁-C₆)-Alkyl stehen, steht
das dadurch gekennzeichnet ist, daß man eine Verbindung der Formel II, 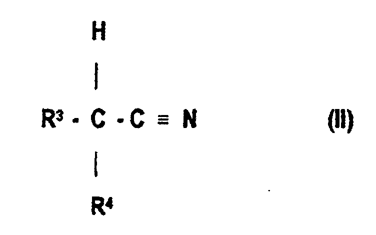 wobei
R³ dieselbe Bedeutung wie in Formel I hat und
R⁴ Wasserstoffatom bedeutet oder dieselbe Bedeutung wie R² in Formel I hat,
gegebenenfalls zuerst in einem organischen Lösungsmittel löst oder ohne Lösungsmittel mit einem Alkylierungsmittel der Formel III,

R¹-X (III)

wobei R¹ dieselbe Bedeutung wie in Formel I hat und
X für Halogen steht oder 2 Reste von R¹ an dem Rest SO₄ gebunden sind oder
mit einem Alkylierungsmittel der Formel IIIa, 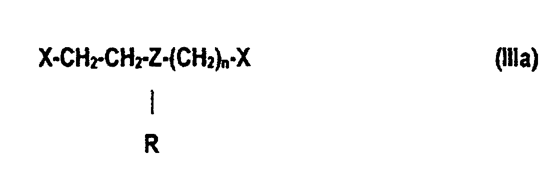 worin Z, X, R und n die obengenannte Bedeutung haben,

in Gegenwart von einem Alkalihydroxid und mindestens einer Verbindung der Formel V und/oder VI 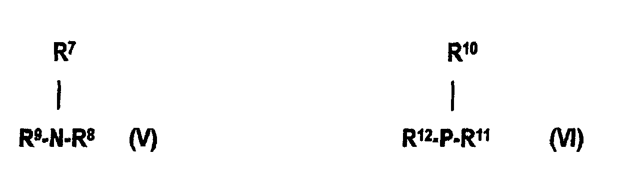 worin
R⁷, R⁸, R⁹, R¹⁰, R¹¹ und R¹² unabhängig voneinander gleich oder
verschieden sind und für (C₁-C₃₀)-Alkyl oder Phenyl stehen, umsetzt

Bevorzugt werden die Verbindungen der Formel I hergestellt, worin
R¹ für
   1. (C₁-C₆)-Alkyl,
   2. (C₁-C₆)-Alkyl, zweifach substituiert durch -O-CH₃ oder
   3. (C₁-C₆)-Alkyl, einfach substituiert durch R⁵-(R⁶)-N-, worin R⁵ und R⁶ gleich oder verschieden sind und Wasserstoffatom oder (C₁-C₃)-Alkyl bedeuten, steht,
R² dieselbe Bedeutung wie R¹ hat oder für Phenyl steht oder
R¹ und R² zusammen mit dem Kohlenstoffatom an das sie gebunden sind und den Resten R³ und -CN eine Verbindung der Formel IVa bilden 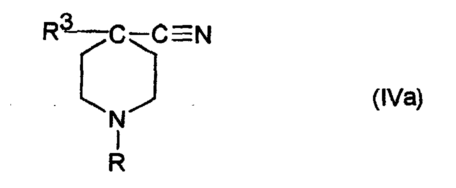 worin R (C₁-C₆)-Alkyl, Benzyl oder Phenyl bedeutet,
R³ für Phenyl, unsubstituiert oder einfach substituiert durch (C₁-C₃)-Alkyl-O-, steht

Vorteilhaft werden die Verbindungen der Formel I hergestellt, worin
R¹ für (C₁-C₃)-Alkyl, (C₁-C₃)-Alkyl, zweifach substituiert durch -O-CH₃ oder -CH(CH₃)-CH₂-N-(CH₃)-CH₃ steht,
R² dieselbe Bedeutung wie R¹ hat oder für Phenyl steht oder
R¹ und R² zusammen mit dem Kohlenstoffatom an das sie gebunden sind und den Resten R³ und -CN einen Rest der Formel IVa bilden, worin R -CH₃ bedeutet,
R³ für Phenyl, unsubstituiert oder einfach substituiert durch -O-CH₃, steht.

Bevorzugt werden nach dem erfindungsgemäßen Verfahren Verbindungen der Formel I hergestellt, wobei R¹ und R² für (C₁-C₆)-Alkyl steht und R³ für Phenyl.

Insbesondere bevorzugt wird Dimethylbenzylcyanid oder 1-Methyl-4-phenyl-piperidin-4-carbonitril hergestellt

Bei der Herstellung der Verbindung der Formel I geht man so vor, daß zunächst das basische Mittel und die Verbindung der Formeln V und/oder VI unter Rühren vorgelegt werden und dann durch Zugabe der Verbindung der Formel II, die gegebenenfalls zuvor in einem organischen Lösungsmittel gelöst wurde, und des Alkylierungsmittels zu einer Verbindung der Formel I umgesetzt wird.

Vorzugsweise verwendet man auf 1 Mol der Verbindung der Formel II von 2,1 Mol bis 2,4 Mol, insbesondere von 2,15 Mol bis 2,25 Mol, des Alkylierungsmittels der Formel III und auf 1 Mol der Verbindung der Formel II verwendet man vorzugsweise von 2,5 Mol bis 4 Mol, insbesondere von 2,8 Mol bis 3,2 Mol, des basischen Mittels.
Vorzugsweise verwendet man auf 100 Gewichtsprozent (Gew.%) der Verbindung der Formel II von 0,5 Gew.% bis 5 Gew.%, insbesondere 1 Gew.% bis 2 Gew.% der Verbindung der Formeln V und/oder VI.

Die Reaktionstemperatur beträgt von 20 °C bis 100 °C, bevorzugt von 30 °C bis 40 °C. Die Reaktionszeit liegt im allgemeinen von 2 bis 10 Stunden.

Liegt das Alkylierungsmittel wie bei Methylchlorid (Chlormethan) gasförmig vor kann auch mit einem Überdruck von bis zu 5 bar gearbeitet werden. Bei der Alkylierungsreaktion mit Dialkylsulfat wird die sich während der Reaktion bildende Methylschwefelsäure durch gegebenenfalls weitere Zugabe von Alkalihydroxid gebunden. Nach Ablauf der Reaktion wird die Verbindung der Formel I isoliert Dazu wird das Gemisch mit Wasser versetzt und die sich dabei bildenden Phasen werden anschließend abgetrennt Die Verbindung der Formel I wird dann aus der organischen Phase gewonnen. Wenn es notwendig erscheint, kann die organische Phase einem Reinigungsverfahren wie z.B. einer Destillation unter vermindertem Druck oder einer Kristallisation aus einem Lösemittel unterzogen werden.

Bevorzugte Alkalihydroxide sind, z. B. Natriumhydroxid, Kaliumhydroxid, Lithiumhydroxid, insbesondere bevorzugt ist Natriumhydroxid.

Bevorzugte Lösemittel sind beispielsweise:
(C₅-C₇)-aliphatische und (C₆-C₈)-cycloaliphatische Kohlenwasserstoffe wie Pentan, 2-Methylbutan, Hexan, 2,2-Dimethylbutan, 2-Methylpentan, 3-Methylpentan, Heptan, Cyclohexan, Methylcyclohexan, 1,2-Dimethylcyclohexan, 1,3-Dimethylcyclohexan; aromatische Kohlenwasserstoffe wie Toluol, Xylole, Ethylbenzol, Isopropylbenzol; aromatische und aliphatische Halogenkohlenwasserstoffe wie Chlorbenzol, Dichlormethan, Dichlorpropan, 1,2-Dichlorethan; Polyether wie Ethylenglykoldibutylether, Dlethylenglykolethyl-tert.-butylether, Polyethylenglykoldimethylether, Polypropylenglykoldibutylether, Polyethylenglykoldimethylether, Polyethylenglykoldiethylether, Polypropylenglykoldiethylether, Polypropylenglykolmethylether; heterocyclische Kohlenwasserstoffe wie N-Methylpyrrolidon, Pyridin; Ether wie Tetrahydrofuran, Dibutylether, Methyl-tert.-butylether sowie Dimethylcarbonat und Dimethylsulfoxid.

Folgende Verbindungen der Formel V und/oder VI kommen in Betracht:
- Trimethylamin, Dimethylethylamin, Triethylamin, Tri-n-propylamin, Triisopropylamin, Tributylamin, Trioctylamin, Tricyclohexylamin, Trihexadecylamin, Diphenylmethylamin, Dimethylbenzylamin, Dibenzylmethylamin, Tribenzylamin, Triphenylamin,
- Trimethylphosphin, Triethylphosphin, Tri-n-propylphosphin, Tributylphosphin, Triisopropylphosphin, Trioctylphosphin, Triphenylphosphin.

Es können auch Mischungen der Verbindungen der Formel V und/oder VI eingesetzt werden. Bevorzugte Verbindungen der Formel V und/oder VI sind (C₃-C₂₄)-Trioctylamin oder (C₃-C₂₄)-Trioctylphosphin. Insbesondere bevorzugte Verbindungen der Formel V und/oder Formel VI sind Trioctylamin, Trioctylphosphin und Triethylamin.

Bevorzugte Alkylierungsmittel sind (C₁-C₆)-Alkylhalogenide wie Alkylchlorid, Alkylbromid, Alkylfluorid oder Alkyljodid, insbesondere Methylchlorid, Ethylchlorid oder Propylchlorid; (C₁-C₆)-Dialkylsulfate wie Dimethyl-, Diethyl-, Dipropyl-, Dibutyl-, Dipentyl- oder Dihexylsulfat oder Di-(2-Chlorethyl)-methylamin. Unter dem Begriff "Halogen" wird Fluor, Chlor, Brom oder Jod verstanden. Unter dem Begriff "Alkyl" oder "Alkenyl" werden Kohlenwasserstoffreste verstanden deren Kohlenstoffkette geradkettig oder verzweigt sind. Cyclische Alkylreste sind beispielsweise 3- bis 6-gliedrige Monocyclen wie Cyclopropyl, Cyclobutyl, Cyclopentyl oder Cyclohexyl. Ferner kann der Alkenylrest mehr als eine Doppelbindung, also auch 2, 3 oder 4 Doppelbindungen enthalten.

Ferner erweist es sich mitunter als vorteilhaft in dem Reaktionsgemisch zusätzlich mindestens eine quartäre Ammoniumverbindung und/oder Phosphoniumverbindung der Formeln VII und VIII 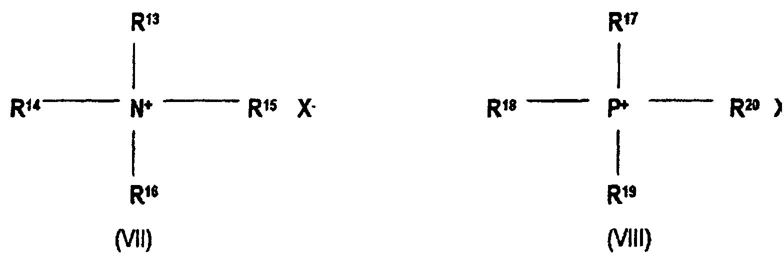 einzusetzen, wobei
R¹³ bis R²⁰ gleich oder verschieden sind und unabhängig voneinander für
   a) (C₁-C₂₀)-Alkyl, geradkettig oder verzweigt,
   b) Benzyl oder
   c) Phenyl stehen und X für Anion steht

Es können auch Mischungen der Verbindungen der Formeln VII und VIII zusätzlich eingesetzt werden. Bevorzugte quartäre Ammonium- oder Phosphoniumverbindungen der Formeln VII und VIII sind Methyltrioctylammoniumchlorid, Methyl-trioctylammoniumhydroxid, Methyl-tricaprylammoniumchlorid, Methyl-tricaprylammoniumhydroxid, Ethyl-trioctylammoniumchlorid, Ethyl-trioctylphosphoniumchlorid und Hexadecyltributylphosphoniumbromid, insbesondere Methyltrioctylammoniumchlorid.

Vorzugsweise verwendet man auf 100 Mol der Verbindung der Formel II von 10 Mol bis 300 Mol, insbesondere von 100 Mol bis 300 Mol der Verbindung der Formeln VII und/oder VIII.

Die Zugabe der Verbindungen der Formeln VII und VIII erfolgt beispielsweise vor der Zugabe der Verbindung der Formel II.

Die Ausgangssubstanzen für die erfindungsgemäße Alkylierungsreaktion sind nach literaturbekannten Verfahren herstellbar, beispielsweise durch Cyanolyse der entsprechenden Halogenverbindungen.

Die Verfahrensprodukte sind gesuchte Verbindungen für die Herstellung einer Vielzahl von Folgeprodukten, z.B. für die Herstellung von antiallergisch wirkenden Arzneimitteln wie 4-[4-[4-(Hydroxydiphenyl)-1-piperidinyl]-1-hydroxybutyl]-α,α-dimethylphenylessigsäure (US 4 254 129).

Vorteilhaft sind die hohen Ausbeuten und die hohe Reinheit der hergestellten Produkte.

### Beispiel 1

### Herstellung von Dimethylbenzylcyanid

In einem Reaktor wurden 1416 g Natronlauge, 33 %ig und 425 g Ätznatron vorgelegt Zu dieser Lösung gab man 6 g Trioctylamin zu, anschließend wurden 400 g Benzylcyanid und 380 g Chlormethan bei 20 °C bis 40 °C unter Rühren bei erhöhtem Druck in diesem Gemisch umgesetzt Nachdem der Innendruck auf unter 0,5 bar gefallen war, wurde der Restdruck entspannt Danach wurden 2000 ml Wasser zugegeben, kurz gerührt, absitzen gelassen und danach wurden die Phasen getrennt Die organische Phase wurde unter verminderten Druck destilliert Man erhielt 485,5 g reines Dimethylbenzylcyanid mit einem Gehalt von mehr als 99 % (ermittelt durch Gaschromatographie (GC)). Das sind 98 % der Theorie bezogen auf eingesetztes Benzylcyanid. Der Gehalt an Monomethylbenzylcyanid und nicht umgesetzten Benzylcyanid lag jeweils unter 0,1 %. Der Gehalt an Verseifungsprodukten wie Phenylessigsäure lag unter der Nachweisgrenze.

| | | |
|---|---|---|
| GC: Trennsäule | | HP1, 25 m lang |
| Temperatur Einspritzblock | | 250 °C |
| Starttemperatur | | 50°C |
| Heizrate | | 10°C/min |
| Endtemperatur | | 250 °C |
| Trägergas | | Helium oder Stickstoff |
| Splitverhäftnis | | 1:100 |
| Detektor | | FID |
| Einspritzmenge | | 3 µl (5 %ige Lösung in Toluol) |
| | | |
| Retentionszeiten | Benzylcyanid | etwa 6,9 min |
| | Monomethylbenzylcyanid | etwa 7,5 min |
| | Dimethylbenzylcyanid | etwa 8,0 min |

### Beispiel 2

### Herstellung von 1-Methyl-4-phenyl-piperidin-4-carbonitril (Dolantinnitril)

In einem Reaktor wurden 1160 g Natronlauge, 33 %ig und 184 g Ätznatron vorgelegt Dieser Lösung setzte man 5 g Trioctylamin und 15 g Methyltrioctylammoniumchlorid zu. Anschließend wurden unter guter Rührung gleichzeitig 117 Benzylcyanid und eine Lösung aus 163,8 g Di-(2-Chlorethyl)-methylamin in 820 g Toluol bei 60 °C bis 80 °C zudosiert Anschließend ließ man noch 2 bis 4 Stunden nachrühren. Danach setzte man 2000 ml Wasser zu, rührte kurz nach und trennte die sich bildenden Phasen voneinander ab. Durch eine zunächst saure, anschließend alkalische Extraktion trennte man das Produkt vom Katalysator (Trioctylamin, Methyltrioctylammoniumchlorid) und organischen Verunreinigungen ab. Von der organischen Phase wurde dann unter verminderten Druck das Toluol abdestilliert. Der Destillationsrückstand wurde anschließend durch Destillation im Hochvakuum bei einem Druck von weniger als 1 mbar weiter gereinigt Als Destillat erhielt man 180 g 1-Methyl-4-phenyl-piperidin-4-carbonitril mit einem Gehalt von mehr als 99 % (ermittelt durch Gaschromatographie (GC)). Das entspricht einer Ausbeute von 90 % der Theorie bezogen auf das eingesetzte Benzylcyanid.

| | | |
|---|---|---|
| GC: Trennsäule | | DB17, 30 m lang |
| Temp. Einspritzblock | | 250 °C |
| Starttemperatur | | 100°C |
| Heizrate | | 10 °C/min |
| Endtemperatur | | 250 °C |
| Trägergas | | Helium |
| Splitverhältnis | | 1:100 |
| Detektor | | FID |
| Einspritzmenge | | 3 µl (5 %ige Lösung in Toluol) |
| | | |
| Retentionszeiten | Benzylcyanid | etwa 9 min |
| | Dolantinnitril | etwa 18 min |

## Patentansprüche

1. Verfahren zur Gewinnung der Verbindung der Formel I 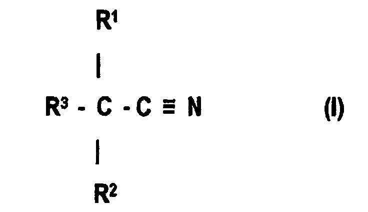 wobei
R¹ für
1. (C₁-C₂₀)-Alkyl,
2. (C₁-C₂₀)-Alkyl, welches ein-, zwei- oder dreifach substituiert ist durch
2.1. (C₃-C₆)-Cycloalkyl,
2.2. -OH,
2.3. (C₁-C₆)-Alkyl-C(O)-O-,
2.4. (C₁-C₆)-Alkyl-O-,
2.5. (C₁-C₆)-Alkyl-O-(C₁-C₄)-Alkyl-O-,
2.6. Halogen,
2.7. -CF₃,
2.8. -CN,
2.9. -NO₂,
2.10. HO-C(O)-,
2.11. (C₁-C₆)-Alkyl-O-C(O)-,
2.12. Methylendioxo,
2.13. R⁵-(R⁶)N-C(O)-, worin R⁵ und R⁶ gleich oder verschieden sind und Wasserstoffatom oder (C₁-C₆)-Alkyl bedeuten,
2.14. R⁵-(R⁶)N-, worin R⁵ und R⁶ gleich oder verschieden sind und Wasserstoffatom oder (C₁-C₆)-Alkyl bedeuten, oder
2.15 Phenyl, welches unsubstituiert oder ein-, zwei- oder dreifach unabhängig voneinander substituiert ist durch (C₁-C₆)-Alkyl oder wie unter 2.1. bis 2.14. beschrieben substituiert ist,
3. (C₂-C₂₀)-Alkenyl oder
4. (C₂-C₂₀)-Alkenyl, welches ein-, zwei- oder dreifach unabhängig voneinander wie unter 2.1. bis 2.15. beschrieben substituiert ist, steht,
R² dieselbe Bedeutung wie R¹ hat oder
für
1. Phenyl oder
2. Phenyl, ein-, zwei- oder dreifach substituiert durch
2.1. (C₁-C₆)-Alkyl, worin die Alkylkette gerade oder verzweigt ist,
2.2. (C₃-C₆)-Cycloalkyl,
2.3. -OH,
2.4. (C₁-C₆)-Alkyl-C(O)-O-,
2.5. (C₁-C₆)-Alkyl-O-,
2.6. (C₁-C₆)-Alkyl-O-(C₁-C₄)-Alkyl-O-,
2.7. Halogen,
2.8. -CF₃,
2.9. -CN,
2.10. -NO₂,
2.11. HO-C(O)-,
2.12. (C₁-C₆)-Alkyl-O-C(O)-,
2.13. Methylendioxo,
2.14. R⁵-(R⁶)N-C(O)-, worin R⁵ und R⁶ gleich oder verschieden sind und Wasserstoffatom oder (C₁-C₆)-Alkyl bedeuten, oder
2.15. R⁵-(R⁶)N-, worin R⁵ und R⁶ gleich oder verschieden sind und Wasserstoffatom oder (C₁-C₆)-Alkyl bedeuten, steht
oder R¹ und R² zusammen mit dem Kohlenstoffatom an das sie gebunden sind und den Resten R³ und -CN eine Verbindung der Formel IV bilden, 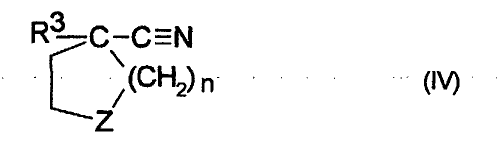 wobei
Z für N-, O- oder S-Atom steht und n 1 oder 2 ist, oder wenn Z für N- oder S-Atom steht, Z unsubstituiert oder durch R substituiert ist, worin R (C₁-C₆)-Alkyl, Benzyl oder Phenyl bedeutet,
R³ für
1. Phenyl oder
2. Phenyl, ein-, zwei- oder dreifach substituiert durch
2.1. (C₁-C₆)-Alkyl, worin die Alkylkette gerade oder verzweigt ist,
2.2. (C₃-C₆)-Cycloalkyl,
2.3. -OH,
2.4. (C₁-C₆)-Alkyl-C(O)-O-,
2.5. (C₁-C₆)-Alkyl-O-,
2.6. (C₁-C₆)-Alkyl-O-(C₁-C₄)-Alkyl-O-,
2.7. Halogen,
2.8. -CF3,
2.9. -CN,
2.10. -NO₂,
2.11. HO-C(O)-,
2.12. (C₁-C₆)-Alkyl-O-C(O)-,
2.13. Methylendioxo,
2.14. R⁵-(R⁶)N-C(O)-, worin R⁵ und R⁶ gleich oder verschieden sind und Wasserstoffatom oder (C₁-C₆)-Alkyl bedeuten, oder
2.15. R⁵-(R⁶)N-, worin R⁵ und R⁶ gleich oder verschieden sind und Wasserstoffatom oder (C₁-C₆)-Alkyl bedeuten, steht,
**dadurch gekennzeichnet, daß** man eine Verbindung der Formel II, 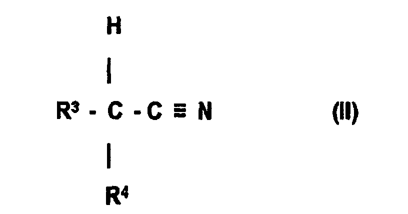 wobei
R³ dieselbe Bedeutung wie in Formel I hat und
R⁴ Wasserstoffatom bedeutet oder dieselbe Bedeutung wie R² in Formel I hat,
gegebenenfalls zuerst in einem organischen Lösungsmittel löst oder ohne Lösungsmittel mit einem Alkylierungsmittel der Formel III,
R¹-X (III)
wobei R¹ dieselbe Bedeutung wie in Formel I hat und X für Halogen steht oder 2 Reste von R¹ an den Rest SO₄ gebunden sind oder
mit einem Alkylierungsmittel der Formel IIIa, 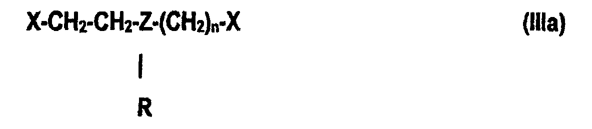 worin Z, X, R und n die obengenannte Bedeutung haben,
in Gegenwart von einem Alkalihydroxid und mindestens einer Verbindung der Formel V und/oder Formel VI 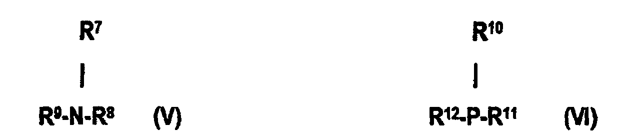 wobei
R⁷, R⁸, R⁹, R¹⁰, R¹¹ und R¹² unabhängig voneinander gleich oder
verschieden sind und für (C₁-C₃₀)-Alkyl oder Phenyl stehen, umsetzt

2. Verfahren gemäß Anspruch 1, **dadurch gekennzeichnet, daß** eine Verbindung der Formel I hergestellt wird, worin
R¹ für
1. (C₁-C₆)-Alkyl,
2. (C₁-C₆)-Alkyl, zweifach substituiert durch -O-CH₃ oder
3. (C₁-C₆)-Alkyl, einfach substituiert durch R⁵-(R⁶)-N-, worin R⁵ und R⁶ gleich oder verschieden sind und Wasserstoffatom oder (C₁-C₃)-Alkyl bedeuten, steht,
R² dieselbe Bedeutung wie R¹ hat oder für Phenyl steht oder
R¹ und R² zusammen mit dem Kohlenstoffatom an das sie gebunden sind und den Resten R³ und -CN eine Verbindung der Formel IVa bilden, 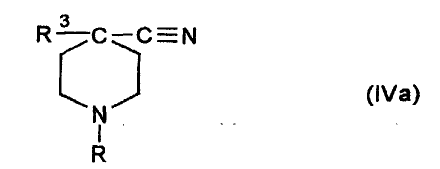 worin R (C₁-C₆)-Alkyl, Benzyl oder Phenyl bedeutet, und
R³ für Phenyl unsubstituiert oder einfach substituiert durch (C₁-C₃)-Alkyl-O- steht.

3. Verfahren gemäß Anspruch 1, **dadurch gekennzeichnet, daß** die Verbindungen der Formel I hergestellt wird, worin
R¹ für (C₁-C₃)-Alkyl, (C₁-C₃)-Alkyl, zweifach substituiert durch -O-CH₃ oder -CH(CH₃)-CH₂-N-(CH₃)-CH₃ steht,
R² dieselbe Bedeutung wie R¹ hat oder für Phenyl steht oder
R¹ und R² zusammen mit dem Kohlenstoffatom an das sie gebunden sind und den Resten R³ und -CN eine Verbindung der Formel IVa bilden, worin R -CH₃ bedeuted,
R³ für Phenyl, unsubstituiert oder einfach substituiert durch -O-CH₃, steht.

4. Verfahren gemäß Anspruch 1, **dadurch gekennzeichnet, daß** man Dimethylbenzylcyanid oder 1-Methyl-4-phenyl-piperidin-4-carbonitril herstellt.

5. Verfahren gemäß einem oder mehreren der Ansprüche 1 bis 4, **dadurch gekennzeichnet, daß** man als Verbindung der Formel V undloder VI eine Verbindung aus der Gruppe Trimethylamin, Dimethylethylamin, Triethylamin, Tri-n-propylamin, Triisopropylamin, Tributylamin, Trioctylamin, Tricyclohexylamin, Trihexadecylamin, Diphenylmethylamin, Dimethylbenzylamin, Dibenzylmethylamin, Tribenzylamin, Triphenylamin, Trimethylphosphin, Triethylphosphin, Tri-n-propylphosphin, Tributylphosphin, Trioctylphosphin, Triisopropylphosphin und Triphenylphosphin einsetzt.

6. Verfahren gemäß Anspruch 5, **dadurch gekennzeichnet, daß** man als Verbindung der Formel V und/oder VI eine Verbindung aus der Gruppe Trioctylamin, Trioctylphosphin und Triethylamin einsetzt.

7. Verfahren gemäß einem oder mehreren der Ansprüche 1 bis 6, **dadurch gekennzeichnet, daß** man als Alkalihydroxid Natriumhydroxid, Kaliumhydroxid, Lithiumhydroxid einsetzt.

8. Verfahren gemäß Anspruch 7, **dadurch gekennzeichnet, daß** man als als Alkalihydroxid Natriumhydroxid einsetzt.

9. Verfahren gemäß einem oder mehreren der Ansprüche 1 bis 7, **dadurch gekennzeichnet, daß** man als Alkylierungsmittel (C₁-C₆)-Alkylhalogenide wie Alkylchlorid, Alkylbromid, Alkylfluorid oder Alkyljodid einsetzt.

10. Verfahren gemäß Anspruch 9, **dadurch gekennzeichnet, daß** man als Alkylierungsmittel Methylchlorid, Ethylchlorid oder Propylchlorid; (C₁-C₆)-Dialkyl-sulfat wie Dimethyl-, Diethyl-, Dipropyl-, Dibutyl-, Dipentyl- oder Dihexylsulfat oder Di-(2-Chlorethyl)-methylamin einsetzt.

11. Verfahren gemäß einem oder mehreren der Ansprüche 1 bis 10, **dadurch gekennzeichnet, daß** man auf 1 Mol der Verbindung der Formel II von 2,1 Moi bis 2,4 Mol des Alkylierungsmittels der Formel III einsetzt und auf 1 Mol der Verbindung der Formel II von 2,5 Mol bis 4 Mol des Alkalihydroxids einsetzt.

12. Verfahren gemäß Anspruch 11, **dadurch gekennzeichnet, daß** man auf 1 Mol der Verbindung der Formel II von von 2,15 Mol bis 2,25 Mol, des Alkylierungsmittels der Formel III einsetzt und auf 1 Mol der Verbindung der Formel II von 2,8 Mol bis 3,2 Mol, des Alkalihydroxids einsetzt.

13. Verfahren gemäß einem oder mehreren der Ansprüche 1 bis 12, **dadurch gekennzeichnet, daß** man auf 100 Gewichtsprozent (Gew.%) der Verbindung der Formel II von 0,5 Gew.% bis 5 Gew.% der Verbindung der Formel V und/oder Formel VI einsetzt.

14. Verfahren gemäß Anspruch 13, **dadurch gekennzeichnet, daß** man auf auf 100 Gewichtsprozent (Gew.%) der Verbindung der Formel II von 1 Gew.% bis 2 Gew.%, der Verbindung der Formel V und/oder Formel VI einsetzt.

15. Verfahren gemäß einem oder mehreren der Ansprüche 1 bis 14, **dadurch gekennzeichnet, daß** man die Alkylierung bei Temperaturen von 20 °C bis 100 °C durchführt.

16. Verfahren gemäß Anspruch 15, **dadurch gekennzeichnet daß** man die Alkylierung bei Temperaturen von 30 °C bis 40 °C durchführt.

17. Verfahren gemäß einem oder mehreren der Ansprüche 1 bis 16, **dadurch gekennzeichnet, daß** man zusätzlich eine Verbindung der Formeln VII und/oder VIII 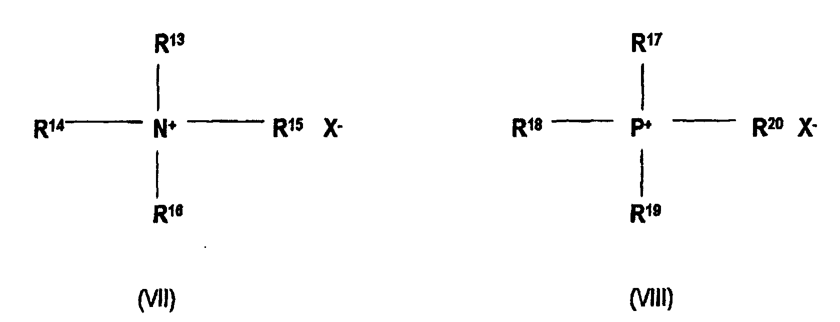 wobei
R¹³ bis R²⁰ gleich oder verschieden sind und unabhängig voneinander für
a) (C₁-C₂₀)-Alkyl, geradkettig oder verzweigt,
b) Benzyl oder
c) Phenyl stehen
und X- für Anion steht, einsetzt.

18. Verfahren gemäß Anspruch 17, **dadurch gekennzeichnet, daß** man als Verbindung der Formeln VII und/oder VIII Methyltrioctylammoniumchlorid, Methyl-trioctylammonlumhydroxid, Methyl-tricaprylammoniumchlorid, Methyl-tricaprylammoniumhydroxid, Ethyl-trioctylammoniumchlorid, Ethyl-trioctylphosphoniumchlorid und Hexadecyltributylphosphoniumbromid einsetzt.

19. Verfahren gemäß Anspruch 18, **dadurch gekennzeichnet, daß** man als Verbindung der Formel VII Methyltrioctylammoniumchlorid einsetzt.

20. Verfahren gemäß der Ansprüche 17 bis 19, **dadurch gekennzeichnet, daß** man auf 100 Mol der Verbindung der Formel II von 10 Mol bis 300 Mol der Verbindung der Formeln VII und/oder VIII einsetzt.

21. Verfahren gemäß Anspruch 20, **dadurch gekennzeichnet, daß** man auf 100 Mol der Verbindung der Formel II von 100 Mol bis 300 Mol der Verbindung der Formeln VII und/oder VIII einsetzt.

22. Verfahren gemäß einem oder mehreren der Ansprüche 1 bis 21, **dadurch gekennzeichnet, daß** die Verbindung der Formel II in einem Lösungsmittel aus der Gruppe (C₅-C₇)-aliphatische und (C₆-C₈)-cycloaliphatische Kohlenwasserstoffe wie Pentan, 2-Methylbutan, Hexan, 2,2-Dimethylbutan, 2-Methylpentan, 3-Methylpentan, Heptan, Cyclohexan, Methylcyclohexan, 1,2-Dimethylcyclohexan, 1,3-Dimethylcyclohexan; aromatische Kohlenwasserstoffe wie Toluol, Xylole, Ethylbenzol, Isopropylbenzol; aromatische und aliphatische Halogenkohlenwasserstoffe wie Chlorbenzol, Dichlormethan, Dichlorpropan, 1,2-Dichlorethan; Polyether wie Ethylenglykoldibutylether, Diethylenglykolethyl-tert.-butylether, Polyethylenglykoldibutylether, Polypropylenglykoldibutylether, Polyethylenglykoldimethylether, Polyethylenglykoldiethylether, Polypropylenglykoldiethylether, Polypropylenglykolmethylether; heterocyclische Kohlenwasserstoffe wie N-Methylpyrrolidon, Pyridin; Ether wie Tetrahydrofuran, Dibutylether, Methyl-tert.-butylether sowie Dimethylcarbonat und Dimethylsulfoxid gelöst wird.

## Claims

1. A process for obtaining the compound of the formula I 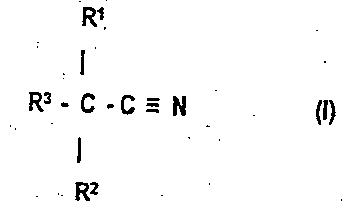 where
R¹ is
1. (C₁-C₂₀)-alkyl,
2. (C₁-C₂₀)-alkyl, which is mono-, di- or trisubstituted by
2.1. (C₃-C₆)-cycloalkyl,
2.2. -OH,
2.3. (C₁-C₆)-alkyl-C(Q)-O-,
2.4. (C₁-C₆)-alkyl-O-,
2.5. (C₁-C₆)-alkyl-O-(C₁-C₄)-alkyl-O-,
2.6. Halogen,
2.7. -CF₃,
2.8. -CN,
2.9. -NO₂,
2.10. HO-C(O)-,
2.11. (C₁-C₆)-alkyl-O-C(O)-,
2.12. Methylenedioxo,
2.13. R⁵-(R⁶)N-C(O)-, in which R⁵ and R⁶ are identical or different and are a hydrogen atom or (C₁-C₆)-alkyl,
2.14. R⁵-(R⁶)N-, in which R⁵ and R⁶ are identical or different and are a hydrogen atom or (C₁-C₆)-alkyl, or
2.15. Phenyl which is unsubstituted or mono-, di- or trisubstituted independently of one another by (C₁-C₆)-alkyl or is substituted as described under 2.1. to 2.14.,
3. (C₂-C₂₀)-alkenyl or
4. (C₂-C₂₀)-alkenyl which is mono-, di- or trisubstituted independently of one another as described under 2.1. to 2.15.,
R² is as defined for R¹ or is
1. Phenyl or
2. Phenyl, mono-, di- or trisubstituted by
2.1. (C₁-C₆)-alkyl, in which the alkyl chain is straight or branched,
2.2. (C₃-C₆)-cycloalkyl,
2.3. -OH,
2.4. (C₁-C₆)-alkyl-C(O)-O-,
2.5. (C₁-C₆)-alkyl-O-,
2.6. (C₁-C₆)-alkyl-O-(C₁-C₄)-alkyl-O-,
2.7. Halogen,
2.8. -CF₃,
2.9. -CN,
2.10. -NO₂,
2.11. HO-C(O)-,
2.12. (C₁-C₆)-alkyl-O-C(O)-,
2.13. Methylenedioxo,
2.14. R⁵-(R⁶)N-C(O)-, in which R⁵ and R⁶ are identical or different and are a hydrogen atom or (C₁-C₆)-alkyl, or
2.15. R⁵-(R⁶)N-, in which R⁵ and R⁶ are identical or different and are a hydrogen atom or (C₁-C₆)-alkyl
or R¹ and R² together with the carbon atom to which they are bonded and the radicals R³ and -CN form a compound of the formula IV, 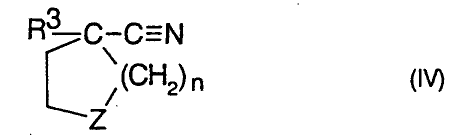 in which Z is a N, O or S atom, and n is 1 or 2, or when Z is a N or S atom, Z is unsubstituted or substituted by R, in which R is (C₁-C₆)-alkyl, benzyl or phenyl,
R³ is
1. Phenyl or
2. Phenyl, mono-, di- or trisubstituted by
2.1. (C₁-C₆)-alkyl, in which the alkyl chain is straight or branched,
2.2. (C₃-C₆)-cycloalkyl,
2.3. -OH,
2.4. (C₁-C₆)-alkyl-C(O)-O-,
2.5. (C₁-C₆)-alkyl-O-,
2.6. (C₁-C₆)-alkyl-O-(C₁-C₄)-alkyl-O-,
2.7. Halogen,
2.8. -CF₃,
2.9. -CN,
2.10. -NO₂,
2.11. HO-C(O)-,
2.12. (C₁-C₆)-alkyl-O-C(O)-,
2.13. Methylenedioxo,
2.14. R⁵-(R⁶)N-C(O)-, in which R⁵ and R⁶ are identical or different and are a hydrogen atom or (C₁-C₆)-alkyl, or
2.15. R⁵-(R⁶)N-, in which R⁵ and R⁶ are identical or different and are a hydrogen atom or (C₁-C₆)-alkyl,
which comprises reacting a compound of the formula II, 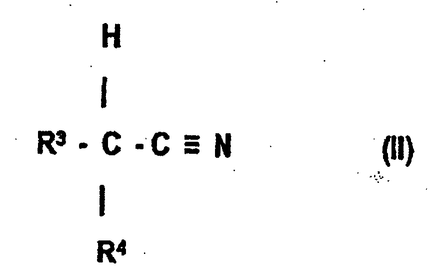
where R³ is as defined in formula I, and
R⁴ is a hydrogen atom or is as defined for R² in formula I,
if necessary firstly dissolved in an organic solvent, or without a solvent, with an alkylating agent of the formula III,
R¹-X (III)
where R¹ is as defined in formula I, and X is halogen
or 2 radicals of R¹ are bonded to the radical SO₄, or
with an alkylating agent of the formula IIIa, 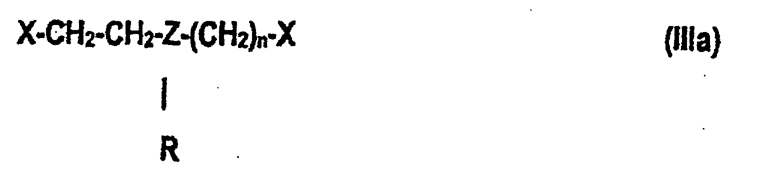 in which Z, X, R and n are as defined above,
in the presence of an alkali metal hydroxide and at least one compound of the formula V and/or formula VI 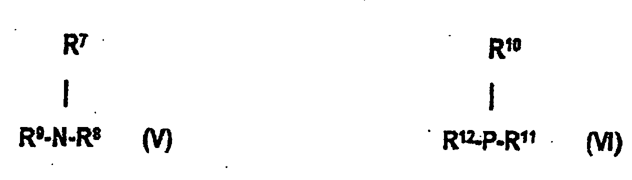
where R⁷, R⁸, R⁹, R¹⁰, R¹¹ and R¹² independently of one another are identical or different
and are (C₁-C₃₀)-alkyl or phenyl.

2. The process as claimed in claim 1, which comprises preparing a compound of the formula I in which
R¹ is
1. (C₁-C₆)-alkyl,
2. (C₁-C₆)-alkyl, disubstituted by -O-CH₃ or
3. (C₁-C₆)-alkyl, monosubstituted by R⁵-(R⁶)-N-, in which R⁵ and R⁶ are identical or different and are a hydrogen atom or (C₁-C₃)-alkyl,
R² is as defined for R¹ or is phenyl, or
R¹ and R² together with the carbon atom to which they are bonded and the radicals R³ and -CN form a compound of the formula IVa, 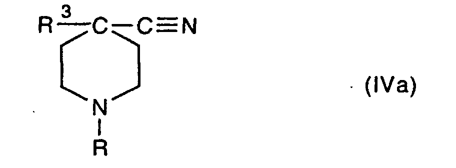 in which R is (C₁-C₆)-alkyl, benzyl or phenyl, and
R³ is phenyl, unsubstituted or monosubstituted by (C₁-C₃)-alkyl-O-.

3. The process as claimed in claim 1, which comprises preparing the compounds of the formula I in which
R¹ is (C₁-C₃)-alkyl, (C₁-C₃)-alkyl, disubstituted by -O-CH₃ or -CH(CH₃)-CH₂-N-(CH₃)-CH₃,
R² is as defined for R¹ or is phenyl, or
R¹ and R² together with the carbon atom to which they are bonded and the radicals R³ and -CN form a compound of the formula IVa where R is -CH₃,
and
R³ is phenyl, unsubstituted or monosubstituted by -O-CH₃.

4. The process as claimed in claim 1, which comprises preparing dimethylbenzyl cyanide or 1-methyl-4-phenylpiperidine-4-carbonitrile.

5. The process as claimed in one or more of claims 1 to 4, wherein the compound of the formula V and/or VI is a compound from the group consisting of trimethylamine, dimethylethylamine, triethylamine, tri-n-propylamine, triisopropylamine, tributylamine, trioctylamine, tricyclohexylamine, trihexadecylamine, diphenylmethylamine, dimethylbenzylamine, dibenzylmethylamine, tribenzylamine, triphenylamine, trimethylphosphine, triethylphosphine, tri-n-propylphosphine, tributylphosphine, trioctylphosphine, triisopropylphosphine and triphenylphosphine.

6. The process as claimed in claim 5, wherein the compound of the formula V and/or VI is a compound from the group consisting of trioctylamine, trioctylphosphine and triethylamine.

7. The process as claimed in one or more of claims 1 to 6, wherein the alkali metal hydroxide is sodium hydroxide, potassium hydroxide and lithium hydroxide.

8. The process as claimed in claim 7, wherein the alkali metal hydroxide is sodium hydroxide.

9. The process as claimed in one or more of claims 1 to 7, wherein the alkylating agent is a (C₁-C₆)-alkyl halide, such as alkyl chloride, alkyl bromide, alkyl fluoride or alkyl iodide.

10. The process as claimed in claim 9, wherein the alkylating agent is methyl chloride, ethyl chloride or propyl chloride; a (C₁-C₆)-dialkyl sulfate, such as dimethyl, diethyl, dipropyl, dibutyl, dipentyl or dihexyl sulfate, or di-(2-chloroethyl)-methylamine.

11. The process as claimed in one or more of claims 1 to 10, wherein, per mole of the compound of the formula II, from 2.1 mol to 2.4 mol of the alkylating agent of the formula III are used, and, per mole of the compound of the formula II, from 2.5 mol to 4 mol of the alkali metal hydroxide are used.

12. The process as claimed in claim 11, wherein, per mole of the compound of the formula II, from 2.15 mol to 2.25 mol of the alkylating agent of the formula III are used, and, per mole of the compound of the formula II, from 2.8 mol to 3.2 mol of the alkali metal hydroxide are used.

13. The process as claimed in one or more of claims 1 to 12, wherein, per 100 percent by weight (% by wt.) of the compound of the formula II, from 0.5% by wt. to 5% by wt. of the compound of the formula V and/or formula VI are used.

14. The process as claimed in claim 13, wherein, per 100 percent by weight (% by wt.) of the compound of the formula II, from 1% by wt. to 2% by wt. of the compound of the formula V and/or formula VI are used.

15. The process as claimed in one or more of claims 1 to 14, wherein the alkylation is carried out at temperatures of from 20°C to 100°C.

16. The process as claimed in claim 15, wherein the alkylation is carried out at temperatures of from 30°C to 40°C.

17. The process as claimed in one or more of claims 1 to 16, which comprises additionally using a compound of the formulae VII and/or VIII 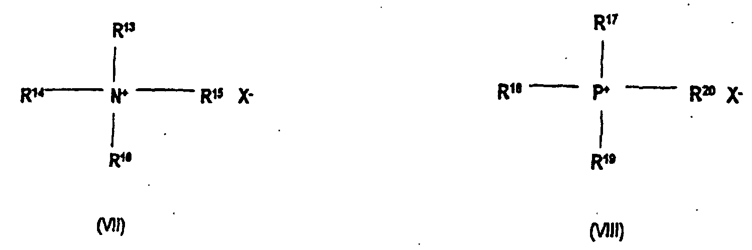 where
R¹³ to R²⁰ are identical or different and independently of one another are
a) (C₁-C₂₀)-alkyl, straight-chain or branched,
b) Benzyl or
c) Phenyl,
and X is an anion.

18. The process as claimed in claim 17, wherein the compound of the formulae VII and/or VIII is methyltrioctylammonium chloride, methyltrioctylammonium hydroxide, methyltricaprylammonium chloride, methyl-tricaprylammonium hydroxide, ethyltrioctylammonium chloride, ethyl-trioctylphosphonium chloride and hexadecyltributylphosphonium bromide.

19. The process as claimed in claim 18, wherein the compound of the formula VII is methyltrioctylammonium chloride.

20. The process as claimed in claims 17 to 19, wherein, per 100 mol of the compound of the formula II, from 10 mol to 300 mol of the compound of the formulae VII and/or VIII are used.

21. The process as claimed in claim 20, wherein, per 100 mol of the compound of the formula II, from 100 mol to 300 mol of the compound of the formulae VII and/or VIII are used.

22. The process as claimed in one or more of claims 1 to 21, wherein the compound of the formula II is dissolved in a solvent from the group consisting of (C₅-C₇)-aliphatic and (C₆-C₈)-cycloaliphatic hydrocarbons, such as pentane, 2-methylbutane, hexane, 2,2-dimethylbutane, 2-methylpentane, 3-methylpentane, heptane, cyclohexane, methylcyclohexane, 1,2-dimethylcyclohexane and 1,3-dimethylcyclohexane; aromatic hydrocarbons, such as toluene, xylenes, ethylbenzene and isoproylbenzene; aromatic and aliphatic halogenated hydrocarbons, such as chlorobenzene, dichloromethane, dichloropropane and 1,2-dichloroethane; polyethers, such as ethylene glycol dibutyl ether, diethylene glycol ethyl tert-butyl ether, polyethylene glycol dibutyl ether, polypropylene glycol dibutyl ether, polyethylene glycol dimethyl ether, polyethylene glycol diethyl ether, polypropylene glycol diethyl ether and polypropylene glycol methyl ether; heterocyclic hydrocarbons, such as N-methylpyrrolidone and pyridine; ethers, such as tetrahydrofuran, dibutyl ether, methyl tert-butyl ether, and also dimethyl carbonate and dimethyl sulfoxide.

## Revendications

1. Procédé pour l'obtention du composé de formule I 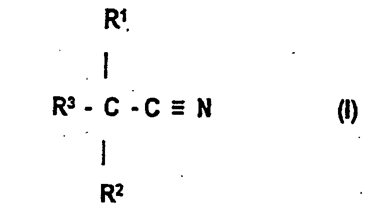 dans laquelle R¹ représente un groupe
1. alkyle en C₁ à C₂₀,
2. alkyle en C₁ à C₂₀, qui est une, deux ou trois fois substitué par
2.1 un groupe cycloalkyle en C₃ à C₆,
2.2 -OH,
2.3 alkyle en C₁ à C₆-C(O)-O-,
2.4 alkyle en C₁ à C₆-O-,
2.5 alkyle en C₁ à C₆-O-alkyle en C₁ à C₄-O-,
2.6 halogène,
2.7 -CF₃,
2.8 -CN,
2.9 -NO₂,
2.10 HO-C(O)-,
2.11 alkyle en C₁ à C₆-O-C(O)-,
2.12 méthylènedioxo,
2.13 R⁵-(R⁶)N-C(O)-, dans laquelle R⁵ et R⁶ sont identiques ou différents et représentent un atome d'hydrogène ou un groupe alkyle en C₁ à C₆,
2.14 R⁵-(R⁶)N-, dans laquelle R⁵ et R⁶ sont identiques ou différents et représentent un atome d'hydrogène ou un groupe alkyle en C₁ à C₆, ou
2.15 phényle, qui est non substitué ou une, deux ou trois fois substitué indépendamment les uns des autres par un groupe alkyle en C₁ à C₆ ou comme décrit en 2.1 à 2.14,
3. alcényle en C₂ à C₂₀ ou
4. alcényle en C₂ à C₂₀, qui est substitué une, deux ou trois fois indépendamment les uns des autres ou comme décrit en 2.1 à 2.15,
R² a la même signification que R¹ ou représente un groupe
1. phényle ou
2. phényle, une, deux ou trois fois substitué par
2.1 un groupe alkyle en C₁ à C₆, dans lequel la chaîne alkyle est linéaire ou ramifiée,
2.2 cycloalkyle en C₃ à C₆,
2.3 -OH,
2.4 alkyle en C₁ à C₆-C(O)-O-,
2.5 alkyle en C₁ à C₆-O-,
2.6 alkyle en C₁ à C₆-O-alkyle en C₁ à C₄-O-,
2.7 halogène,
2.8 -CF₃,
2.9 -CN,
2.10 -NO₂,
2.11 HO-C(O)-,
2.12 alkyle en C₁ à C₆-O-C(O)-,
2.13 méthylènedioxo,
2.14 R⁵-(R⁶)N-C(O)-, dans laquelle R⁵ et R⁶ sont identiques ou différents et représentent un atome d'hydrogène ou un groupe alkyle en C₁ à C₆, ou
2.15 R⁵-(R⁶)N-, dans laquelle R⁵ et R⁶ sont identiques ou différents et représentent un atome d'hydrogène ou un groupe alkyle en C₁ à C₆,
ou R¹ et R² forment ensemble avec l'atome de carbone auquel ils sont liés et les groupes R³ et -CN un composé de formule IV, 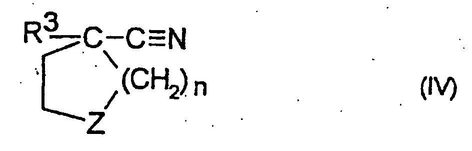 dans laquelle
Z représente un atome N, O ou S et n est 1 ou 2, ou lorsque Z représente un atome N ou S, Z est non substitué ou substitué par R, dans laquelle R représente un groupe alkyle en C₁ à C₆, benzyle ou phényle,
R³ représente un groupe
1. phényle ou
2. phényle, une, deux ou trois fois substitué par
2.1 un groupe alkyle en C₁ à C₆, dans lequel la chaîne alkyle est linéaire ou ramifiée,
2.2 cycloalkyle en C₃ à C₆,
2.3 -OH,
2.4 alkyle en C₁ à C₆-C(O)-O-,
2.5 alkyle en C₁ à C₆-O-,
2.6 alkyle en C₁ à C₆-O-alkyle en C₁ à C₄-O-,
2.7 halogène,
2.8 -CF₃,
2.9 -CN,
2.10 -NO₂,
2.11 HO-C(O)-,
2.12 alkyle en C₁ à C₆-O-C(O)-,
2.13 méthylènedioxo,
2.14 R⁵-(R⁶)N-C(O)-, dans laquelle R⁵ et R⁶ sont identiques ou différents et représentent un atome d'hydrogène ou un groupe alkyle en C₁ à C₆, ou
2.15 R⁵-(R⁶)N-, dans laquelle R⁵ et R⁶ sont identiques ou différents et représentent un atome d'hydrogène ou un groupe alkyle en C₁ à C₆,
**caractérisé en ce qu'**on dissout un composé de formule II, 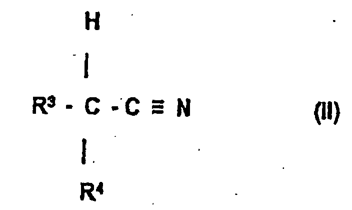 dans laquelle
R³ a la même signification qu'à la formule I et
R⁴ représente un atome d'hydrogène ou a la même signification que R² dans la formule I,
éventuellement d'abord dans un solvant organique ou sans solvant et on le met à réagir avec un agent d'alkylation de formule III,
R¹-X (III)
dans laquelle R¹ a la même signification que dans la formule I et X représente un halogène ou 2 groupes de R¹ sont liés au groupe SO₄ ou
avec un agent d'alkylation de formule IIIa, 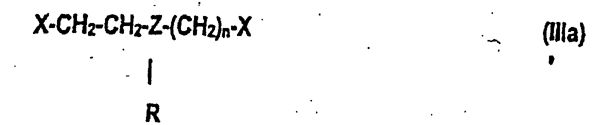 dans laquelle Z, X, R et n ont la signification mentionnée ci-dessus,
en présente d'un hydroxyde alcalin et d'au moins un composé de formule V et/ou de formule VI 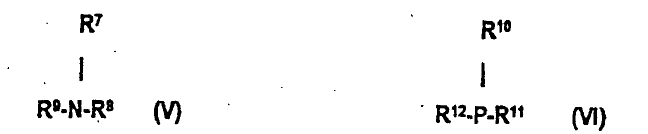 dans lesquelles R⁷, R⁸, R⁹, R¹⁰, R¹¹ et R¹² sont indépendamment les uns des autres identiques ou différents et représentent un groupe alkyle en C₁ à C₃₀ ou phényle.

2. Procédé selon la revendication 1, **caractérisé en ce qu'**on prépare un composé de formule I, dans laquelle
R¹ représente
1. un groupe alkyle en C₁ à C₆,
2. un groupe alkyle en C₁ à C₆, deux fois substitué par -O-CH₃ ou
3. un groupe alkyle en C₁ à C₆, une fois substitué par R⁵-(R⁶)-N-, dans laquelle R⁵ et R⁶ sont identiques ou différents et représentent un atome d'hydrogène ou un groupe alkyle en C₁ à C₃,
R² a la même signification que R¹ ou représente un groupe phényle ou
R¹ et R² forment ensemble avec l'atome de carbone auquel ils sont liés et les groupes R³ et -CN un composé de formule IVa, 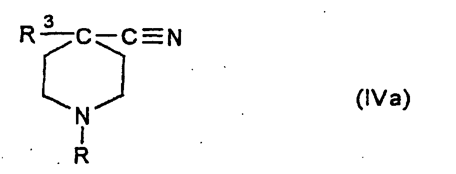 dans laquelle R représente un groupe alkyle en C₁ à C₆, benzyle ou phényle, et
R³ représente un groupe phényle non substitué ou substitué par alkyle en C₁ à C₃-O-.

3. Procédé selon la revendication 1, **caractérisé en ce qu'**on prépare les composés de formule I, dans laquelle
R¹ représente un groupe alkyle en C₁ à C₃, alkyle en C₁ à C₃, deux fois substitué par -O-CH₃ ou -CH(CH₃)-CH₂-N-(CH₃)-CH₃,
R² a la même signification que R¹ ou représente un phényle ou
R¹ et R² forment ensemble avec l'atome de carbone auquel ils sont liés et les groupes R³ et -CN un composé de formule IVa, dans laquelle R représente -CH₃,
R³ représente un groupe phényle, non substitué ou une fois substitué par -O-CH₃.

4. Procédé selon la revendication 1, **caractérisé en ce qu'**on prépare le cyanure de diméthylbenzyle ou le 1-méthyl-4-phényl-pipérinine-4-carbonitrile.

5. Procédé selon une ou plusieurs des revendications 1 à 4, **caractérisé en ce qu'**on utilise comme composé de formule V et/ou VI un composé dans le groupe triméthylamine, diméthyléthylamine, triéthylamine, tri-n-propylamine, triisopropylamine, tributylamine, trioctylamine, tricyclohexylamine, trihexadécylamine, diphénylméthylamine, diméthylbenzylamine, dibenzylméthylamine, tribenzylamine, triphénylamine, triméthylphosphine, triéthylphosphine, tri-n-propylphosphine, tributylphosphine, trioctylphosphine, triisopropylphosphine et triphénylphosphine.

6. Procédé selon la revendication 5, **caractérisé en ce qu'**on utilise comme composé de formule V et/ou VI un composé dans le groupe trioctylamine, trioctylphosphine et triéthylamine.

7. Procédé selon une ou plusieurs des revendications 1 à 6, **caractérisé en ce qu'**on utilise comme hydroxyde de métal alcalin l'hydroxyde de sodium, l'hydroxyde de potassium, l'hydroxyde de lithium.

8. Procédé selon la revendication 7, **caractérisé en ce qu'**on utilise comme hydroxyde de métal alcalin l'hydroxyde de sodium.

9. Procédé selon une ou plusieurs des revendications 1 à 7, **caractérisé en ce qu'**on utilise comme agent d'alkylation des halogénures d'alkyle en C₁ à C₆ comme le chlorure d'alkyle, le bromure d'alkyle, le fluorure d'alkyle ou l'iodure d'alkyle.

10. Procédé selon la revendication 9, **caractérisé en ce qu'**on utilise comme agent d'alkylation le chlorure de méthyle, le chlorure d'éthyle ou le chlorure de propyle ; le sulfate de dialkyle en C₁ à C₆ comme le sulfate de diméthyle, diéthyle, dipropyle, dibutyle, dipentyle ou dihexyle ou la di-(2-chloroéthyl)-méthylamine.

11. Procédé selon une ou plusieurs des revendications 1 à 10, **caractérisé en ce qu'**on utilise pour 1 mol du composé de formule II de 2,1 mol à 2,4 mol de l'agent d'alkylation de formule III et pour 1 mol du composé de formule II de 2,5 à 4 mol de l'hydroxyde de métal alcalin.

12. Procédé selon la revendication 11, **caractérisé en ce qu'**on utilise pour 1 mol du composé de formule II de 2,15 mol à 2,25 mol, de l'agent d'alkylation de formule III et pour 1 mol du composé de formule II de 2,8 mol à 3,2 mol de l'hydroxyde de métal alcalin.

13. Procédé selon une ou plusieurs des revendications 1 à 12, **caractérisé en ce qu'**on utilise pour 100 pour cent en poids (% en poids) du composé de formule II de 0,5 % en poids à 5 % en poids du composé de formule V et/ou de formule VI.

14. Procédé selon la revendication 13, **caractérisé en ce qu'**on utilise pour 100 pour cent en poids (% en poids) du composé de formule II de 1 % en poids à 2 % en poids du composé de formule V et/ou de formule VI.

15. Procédé selon une ou plusieurs des revendications 1 à 14, **caractérisé en ce qu'**on réalise l'alkylation à des températures de 20°C à 100°C.

16. Procédé selon la revendication 15, **caractérisé en ce qu'**on réalise l'alkylation à des températures de 30°C à 40°C.

17. Procédé selon une ou plusieurs des revendications 1 à 16, caractérisé en qu'on utilise en plus un composé des formules VII et/ou VIII 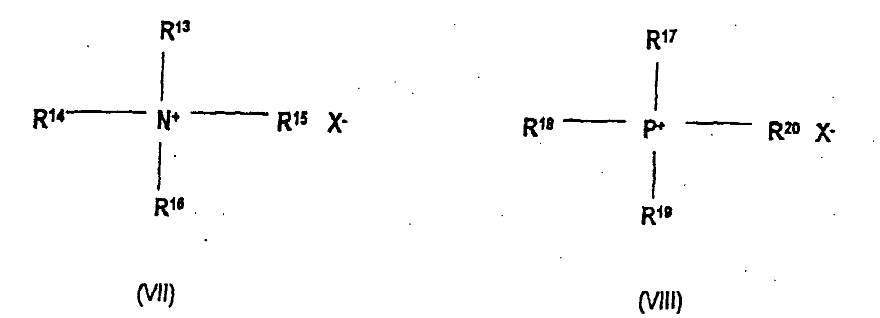
dans lesquelles R¹³ à R²⁰ sont identiques ou différents et représentent indépendamment les uns des autres
a) un groupe alkyle en C₁ à C₂₀, à chaîne linéaire ou ramifiée,
b) benzyle ou
c) phényle
et X⁻ représente un anion.

18. Procédé selon la revendication 17, **caractérisé en ce qu'**on utilise comme composé des formules VII et/ou VIII le chlorure de méthyltrioctylammonium, l'hydroxyde de méthyl-trioctylammonium, le chlorure de méthyltricaprylammonium, l'hydroxyde de méthyl-tricaprylammonium, le chlorure d'éthyl-trioctylammonium, le chlorure d'éthyl-trioctylphosphonium et le bromure d'hexadécyltributylphosphonium.

19. Procédé selon la revendication 18, **caractérisé en ce qu'**on utilise comme composé de formule VII le chlorure de méthyltrioctylammonium.

20. Procédé selon les revendications 17 à 19, **caractérisé en ce qu'**on utilise pour 100 mol du composé de formule II de 10 mol à 300 mol du composé des formules VII et/ou VIII.

21. Procédé selon la revendication 20, **caractérisé en ce qu'**on utilise pour 100 mol du composé de formule II de 100 mol à 300 mol du composé des formules VII et/ou VIII.

22. Procédé selon une ou plusieurs des revendications 1 à 21, caractérisé en que le composé de formule II est dissous dans un solvant dans le groupe des hydrocarbures aliphatiques en C₅ à C₇ et cycloaliphatiques en C₆ à C₈ comme le pentane, le 2-méthylbutane, l'hexane, le 2,2-diméthylbutane, le 2-méthylpentane, le 3-méthylpentane, l'heptane, le cyclohexane, le méthylcyclohexane, le 1,2-diméthylcyclohexane, le 1,3-diméthylcyclohexane; des hydrocarbures aromatiques comme le toluène, les xylènes, l'éthylbenzène, l'isopropylbenzène ; des hydrocarbures halogénés aromatiques et aliphatiques comme le chlorobenzène, le dichlorométhane, le dichloropropane, le 1,2-dichlorométhane, le 1,2-dichloropropane, le 1,2-dichloroéthane ; des polyéthers comme l'éthylèneglycoldibutyléther, le diéthylèneglycol-tert.-butyléther, le polyéthylèneglycoldibutyléther, le polypropylèneglycoldibutyléther, le polyéthylèneglycoldiméthyléther, le polyéthylèneglycoldiéthyléther, le polypropylèneglycoldiéthyléther, le polypropylèneglycolméthyléther ; des hydrocarbures hétérocycliques comme la N-méthylpyrrolidone, la pyridine ; des éthers comme le tétrahydrofurane, le dibutyléther, le méthyl-tert.-butyléther ainsi que le carbonate de diméthyle et le diméthylsulfoxyde.
